(19)

**Europäisches Patentamt**
**European Patent Office**
**Office européen des brevets**

(11) **EP 4 654 212 A1**

(12) **EUROPEAN PATENT APPLICATION**

(43) Date of publication:
**26.11.2025   Bulletin 2025/48**

(21) Application number: **25174880.2**

(22) Date of filing: **07.05.2025**

(51) International Patent Classification (IPC):
***G16H 40/40*** (2018.01)

(52) Cooperative Patent Classification (CPC):
**G16H 40/40;** A61B 90/70; A61L 2202/24;
G16H 40/20

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **23.05.2024   DE 102024114528**

(71) Applicant: **OLYMPUS WINTER & IBE GMBH
22045 Hamburg (DE)**

(72) Inventor: **Jaskola, Sascha
22767 Hamburg (DE)**

(74) Representative: **Noack, Andreas
c/o Stolmár & Partner
Neuer Wall 71
20354 Hamburg (DE)**

(54) **METHOD OF ESTIMATING A RISK OF CROSS-CONTAMINATION, AND ENDOSCOPE PROVISIONING SYSTEM**

(57)     Provided is a computer-implemented method of estimating a risk that a medical instrument has been cross-contaminated with a problematic pathogen, comprising: obtaining data identifying a plurality of medical instruments provided in a facility; obtaining data representing use of some or all of the plurality of medical instruments in a plurality of first procedures on a plurality of first patients; storing in a database, for each of the used medical instruments, first data identifying the patient on which the medical instrument has been used; reprocessing the used medical instruments; - storing in the database, for each of the reprocessed medical instruments, second data identifying at least one reprocessing modality; determining, for each of the plurality of first patients, a first indicator representing whether a respective first patient carries a problematic pathogen; defining, for each reprocessing modality, a first cross-contamination probability factor representing the probability that a problematic pathogen is transferred from a first medical instrument carrying a problematic pathogen to a second medical instrument not carrying the problematic pathogen while the first and second medical instruments share the same reprocessing modality; and combining the first indicators, the first data, the second data, and the first cross-contamination probability factors to determine, for each of the reprocessed medical instruments, a contamination probability that the respective medical instrument carries the problematic pathogen.

Further provided is a medical instrument provisioning system.

Fig. 3

**Description**

<u>Field</u>

The present disclosure relates to a computer-implemented method of estimating a cross-contamination risk of medical instruments, and to an endoscope provisioning system implementing such method.

<u>Background</u>

In modern medicine, diagnostic and therapeutic interventions often involve use of sophisticated medical instruments like endoscopes, electrosurgical devices, and the like, which are complex and expensive. Such medical instruments are therefore often designed as reusable instruments, which can be reprocessed after each use before being used on another patient. Reprocessing may involve cleaning, disinfection, sterilization, and drying of the medical devices.
During reprocessing, the medical devices undergo several separate reprocessing steps in different reprocessing modalities, which may include manual precleaning, automatic reprocessing in endoscope reprocessing machines, drying in drying cabinets, and storage in storage cabinets. Between individual reprocessing steps, medical instruments may need to be transported from one reprocessing modality to another, using transport modalities. Within this disclosure, transport modalities like trays, carts, or the like are also considered reprocessing modalities.
In some of the reprocessing modalities, medical instruments may get in contact with each other, or may get in contact with surfaces or ingredients which have previously been in contact with other medical instruments.
In recent time, awareness has grown that reuse of medical instruments bears a residual risk of cross-contamination, where pathogens may be transferred from one patient to another patient through a medical instrument used on both patients. Such cross-contamination will hereinafter be referred to as vertical cross-contamination.
While cross-contamination is generally undesirable, it can be particularly harmful if problematic pathogens are involved. The term "problematic pathogen" as used herein is supposed to cover pathogens being highly contagious, resistant to one or more antibiotics, causing severe and/or uncurable diseases, or a combination thereof. Examples of problematic pathogens are: multiple-resistant Staphylococcus Aureus (MRSA), HIV, Ebola virus, Hepatitis virus, Prions, etc.
While vertical cross-contamination can, if undetected, can cause significant harm, it can be handled appropriately in most cases. If it is known that a particular patient carries a problematic pathogen, any medical instrument used on that patient may afterwards receive a specific reprocessing treatment targeted at the particular problematic pathogen, or may be discarded completely. If it is only later discovered that a particular patient carries a problematic pathogen, log data may be used to identify other patients who have been treated with the same medical instrument as the patient carrying the problematic pathogen, and appropriate countermeasures may be applied.
However, in addition to vertical cross-contamination described above, it is also possible that problematic pathogens are transferred from one medical instrument to another medical instrument while both medical instruments are reprocessed or transported in the same reprocessing modality, or if both medical instruments get in contact with the same surface or ingredient one after the other. Such cross-contamination may be referred to as horizontal or lateral cross-contamination.
It would be desirable to provide methods for managing the risk of cross-contamination of medical instruments with problematic pathogens.

<u>Summary of the disclosure</u>

The present disclosure provides a computer-implemented method of estimating a risk that a medical instrument has been cross-contaminated with a problematic pathogen, comprising obtaining data identifying a plurality of medical instruments provided in a facility; obtaining data representing use of some or all of the plurality of medical instruments in a plurality of first procedures on a plurality of first patients; storing in a database, for each of the used medical instruments, first data identifying the patient on which the medical instrument has been used; reprocessing the used medical instruments; storing in the database, for each of the reprocessed medical instruments, second data identifying at least one reprocessing modality selected from: a transport device used to transport the medical device before, during, or after reprocessing, a pre-cleaning station in which the medical device has been precleaned, an automatic reprocessing machine in which the medical device has been reprocessed, a drying cabinet in which the medical instrument has been dried, a storage cabinet in which the medical instrument has been stored, and a person handling the medical instrument during reprocessing; determining, for each of the plurality of first patients, a first indicator representing whether a respective first patient carries a problematic pathogen; defining, for each reprocessing modality, a first cross-contamination probability factor representing the probability that a problematic pathogen is transferred from a first medical instrument carrying a problematic pathogen to a second medical instrument not carrying the problematic pathogen while the first and second medical instruments share the same reprocessing modality; and combining the first indicators, the first data, the second data, and the first cross-contamination probability factors to determine, for each of the reprocessed medical instruments, a contamination probability that the respective medical instrument carries the problematic pathogen.

The first indicator may be a binary indicator. In some embodiments, each patient may be assigned a binary flag normally set to zero, but may be set to one if the patient has been diagnosed to carry a problematic pathogen. In some embodiments, each patient may be assigned an array or vector of flags, wherein each flag in the vector of flags indicates the infection of the patient with a particular pathogen.

In some embodiments, the method may further comprise defining, for each reprocessing modality, a reduction factor defining the reduction of a potential load of problematic pathogens while a medical instrument is treated in the respective reprocessing modality; and using the respective reduction factors when determining the contamination probability.

In some embodiments, the method may further comprise defining a first threshold risk level; determining, for each of the used medical instruments, whether the contamination probability exceeds the threshold risk level; and excluding each medical instrument, for which the contamination probability exceeds the threshold risk level, from further use. Various factors may be considered when defining the threshold risk level. In a very conservative approach, the threshold risk level may be set to zero, so that any instrument with a contamination risk greater than zero will be excluded from further use. Other approaches may appreciate that other channels of contamination exist in a given environment, like using the same waiting rooms, air conditioning systems, sanitary installations, and the like. In such approaches, the threshold risk level may be set to a level matching the risk of contamination through such other channels. In even further approaches, at least for problematic pathogens not causing uncurable or lethal diseases, replacement costs of the affected medical instrument and expected costs of treatment of a patient after cross-contamination may be taken into account.

In some embodiments, the method my further comprise obtaining data representing the reuse of some of the medical instruments in a plurality of second procedures on a plurality of second patients; storing in the database, for each of the reused medical instruments, third data identifying the patient on which the medical instrument has been reused; updating, for each of the plurality of first patients, the first indicator representing whether a respective first patient carries a problematic pathogen; combining the updated first binary indicators, the first data, the second data, and the cross-contamination probability factors to determine, for each of the reused medical instruments, an updated contamination probability at the time of reuse.

Herein, it is taken into account that an infection with a problematic pathogen may be detected only after some time. As an example, examination of a sample in a laboratory for detection of problematic pathogens may take some time, while reprocessing and reusing of medical instruments is continued. In other examples, symptoms of an infection with a problematic pathogen may only show after a certain incubation time, while reprocessing and reusing of medical instruments is continued. The method may allow for retroactively updating contamination risk information after an infection with a problematic pathogen surfaces.

The method may further comprise generating an alert if, for at least one of the reused medical instruments, the updated contamination probability at the time of reuse exceeds the threshold risk level. The alert may be used to trigger appropriate countermeasures to alleviate the effects of the problematic pathogen for the second patient.

In some embodiments, the method may further include defining, for a plurality of procedures offered in a facility, a second cross-contamination probability factor representing the probability that a problematic pathogen is transferred from a first medical instrument carrying a problematic pathogen to a second medical instrument not carrying the problematic pathogen while the first and second medical instruments are used together in the respective procedure; reprocessing the reused medical instruments; storing in the database, for each of the reprocessed reused medical instruments, fourth data identifying at least one reprocessing modality used on the respective reused instrument; and combining the updated first binary indicators, the first to fourth data, and the first and second cross-contamination probability factors to determine, for each of the reused medical instruments, a further updated contamination probability. It may thus by taken into account that lateral cross-contamination may not only occur during reprocessing, but only during common use of the medical instruments.

In some embodiments, the disclosure provides a medical instrument provisioning system, comprising: a plurality of medical instruments; a plurality of reprocessing modalities; and a computer comprising a processor, a memory element, and associated hardware;

wherein machine-readable instructions are stored in the memory element for reading and execution by the processor, and wherein the machine-readable instructions cause the processor to execute a method according to the description above.

Brief description of the drawings

In the following, exemplary embodiments of this disclosure are further explained at hand of exemplary drawings, which are not necessarily drawn to scale. The examples and drawings are only provided for a better understanding of the concepts described herein, and are not intended to limit the scope of the disclosure in any way, unless explicitly stated.

Fig. 1 shows a provisioning system for medical instruments;
Fig. 2 shows a schematic view of a computer;
Fig. 3 shows a method according to the disclosure;
Fig. 4 shows an aspect of a method according to the disclosure.

Detailed description

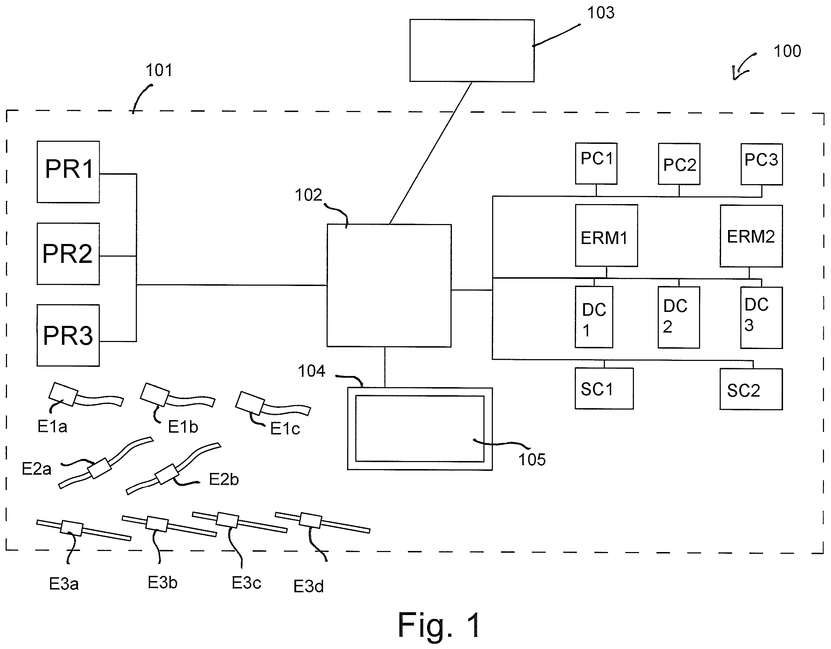

Fig. 1 shows a provisioning system 100 for endoscopes in an exemplary application environment 101, which may be an endoscopy office or a hospital. The application environment comprises several procedure rooms PR1, PR2, PR3. The number of procedure rooms may vary. In the procedure rooms PR1, PR2, PR3, several examination procedures or therapeutic procedures may be performed.

The application environment further comprises a reprocessing facility for reprocessing used medical instruments. The reprocessing facility comprises manual precleaning stations PC1, PC2, PC3, endoscope reprocessing machines ERM1, ERM2, Drying cabinets DC1, DC2, DC3, and storage cabinets SC1, SC2. A plurality of transport modalities like trays, carts, and the like may also be provided in the application environment, but are not shown in Fig. 1.

For performing medical procedures in the procedure rooms PR1, PR2, PR3, a plurality of medical instruments are provided. In the shown example, the medical instruments comprise endoscopes E1a, E1b, E1c of a first type, endoscopes E2a, E2b of a second type, and endoscopes of a third type E3a, E3b, E3c, E3d of a third type. Besides the endoscopes shown in Fig. 1, medical instruments may also include surgical instruments and electrosurgical instruments like forceps, scissors, scalpels, and the like.

The medical instrument provisioning system further comprises a computer 102, which may be a standard or medical grade personal computer. The computer 102 comprises a processor, a memory, and associated hardware (not shown in Fig. 1). The memory of computer 102 stores machine readable instructions, like software, which can be executed by the processor. The computer 102 with the software is configured to receive and process information concerning executed and planned interventions and reprocessing procedures, and the status of the medical instruments provided in the application environment.

Information regarding executed and planned procedures, including patient data, may be provided to the computer 102 from a hospital or office management system 103 though an appropriate interface, which may e.g. use the DICOM standard. Information regarding reprocessing procedures is usually provided by the endoscope reprocessing machines. The computer 102 may further be connected to a display device 104 showing a graphical user interface 105 thereon. The graphical user interface 105 may visualize data received and/or processed by computer 102.

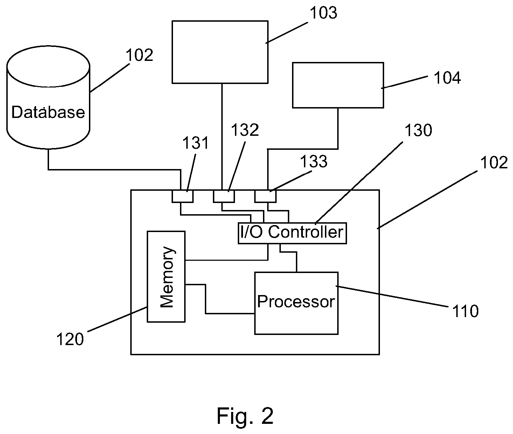

Fig. 2 shows a schematic view of computer 102. The computer comprises a processor 110, which may be a single- or multicore central processing unit (CPU), a graphical processing unit (GPU), a vector processor, or any other suitable processor. The computer 102 further comprises a memory 120 connected to the processor 110. The memory 120 may include random access memory (RAM) and/or read-only memory (ROM). RAM may be implemented using static RAM (SRAM) or dynamic RAM (DRAM). DRAM usually provides for faster access times than SRAM, but requires constant refreshing in order to maintain data. ROM may be implemented as programmable ROM (PROM), erasable programmable ROM (EPROM), or electrically erasable programmable ROM (EEPROM). Memory 120 may further include FLASH memory and/or magnetic, optical, and/or magneto-optical hard drives.

The computer 102 further comprises an input-output (I/O) controller 130 for handling communication between the processor 110 and the outside world. Tie I/O controller 130 offers interfaces 131, 132, 133 for connecting external devices. Interface 131 connects to a database 140, interface 132 connects to the management system 103, and interface 133 connects to the display 104. The I/O controller 130 may be directly connected to the memory 120 to provide direct memory access (DMA) to selected external devices.

The database 140 stores information regarding the medical instruments available in the application environment, planned and executed interventions and reprocessing procedures, and patient data.

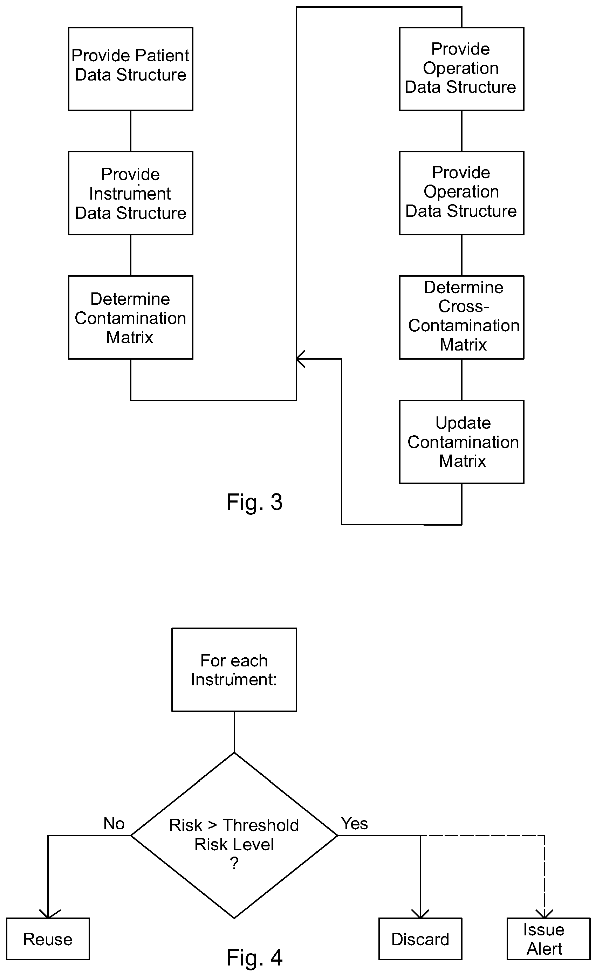

In the following, one embodiment of a data processing method to be executed by processor 110 is described at hand of Fig. 3.

First, a patient data structure is provided representing a plurality of first patients undergoing a procedure in one of the procedure rooms PR1, PR2, PR3. For each patient, a data element is provided indicating whether the respective patient is carrying a problematic pathogen. The patient data structure may be one-dimensional with a single data element for each patient. The data element may be a binary element like a flag $p_x$. In other embodiments, the patient data element may be two-dimensional, wherein a vector of flags $p_{x,z}$ is provided for each patient, and each flag may represent infection with a particular problematic pathogen.

Next, an instrument data structure is provided, which is a two-dimensional matrix. Each data element $I_{x,y}$ of the instrument data structure represents whether an instrument Y has been used on patient X. As each instrument is only used on a single patient, the elements I of each column of the instrument data structure are binary, and add up to 1.

To determine which instrument has potentially been contaminated with a problematic pathogen in the procedure, the patient data structure and the instrument data structure are folded according to the formula $C_{y(,z)} = \sum_x (p_{x(,z)} * I_{x,y})$ to receive a contamination matrix, wherein each data element $C_{y(,z)}$ of the contamination matrix represents an indication that instrument Y has potentially be contaminated. If the patient data structure is two-dimensional, the index z indicates different problematic pathogens.

Next, for each transport or reprocessing step applied to any of the instruments, an operation data structure is provided. The operation data structure is two-dimensional and has one column for each modality used in the step, and one row for each medical instrument. Each data element $O_{y,j}$ of the operation data structure indicates whether instrument Y has been processed or transported by modality j. Further, each individual modality is assigned a cross-contamination probability $K_j$. Again, different pathogens z may be considered by extending the cross-contamination probability to two dimensions $K_{j,(z)}$.

The cross-contamination probabilities $K_{j(z)}$ may be determined at least in part based on empirical data from known cross-contamination events, theoretical considerations, or a combination thereof.

The contamination matrix can then be folded with the operation data structure to obtain a cross-contamination matrix, whereas the folding equation is:

$$CC_{y(,z)} = \sum_j \left( O_{y,j} * \left( 1 - \prod_y \left( 1 - K_{j(,z)} * C_{y(,z)} \right) \right) \right)$$

In the above equation, the combined contamination probability in successive modalities is determined by calculating the inverse probability (1-p) for each modality, multiplying the inverse probabilities, and then again inverting the product.

The cross-contamination matrix can then be used to update the contamination matrix in a similar way: $C'_{y(,z)} = 1 - ((1 - C_{y(,z)}) * (1 - CC_{y(,z)}))$

The previous step can be repeated for each step of a reprocessing procedure. For active reprocessing steps, a pathogen load reduction factor achieved by the respective step can either be applied separately, or can be accounted for when determining the cross-contamination probability of respective reprocessing modalities.

After all reprocessing steps have been considered, the updated contamination matrix shows the risk $C'_{y(,z)}$ that a respective instrument Y has been cross-contamined with pathogen Z during reprocessing.

As shown in Fig. 4, the updated risk $C'_{y(,z)}$ may be compared with a threshold risk level $T_{(z)}$, to determine whether instrument Y can be used for further procedures, or should be discarded. A threshold risk level T can vary depending on the type of pathogen Z. In cases where the initial contamination with a problematic pathogen has been detected after affected medical instruments have been reused on other patients, an alert may be issued to trigger possible countermeasures, which may mitigate the infection risk for the later patient.

In case of multiple cycles of reprocessing and reuse of instruments before a problematic pathogen is detected, cross-contamination can also occur while two medical instruments are used within the same procedure. This can be accounted for by providing a procedure data structure, resembling the operation data structure described above, representing which instruments have been used in which procedures, and applying the same folding operations for determining a cross-contamination matrix as described above.

**Claims**

1. A computer-implemented method of estimating a risk that a medical instrument has been cross-contaminated with a problematic pathogen, comprising:

   - obtaining data identifying a plurality of medical instruments provided in a facility;
   - obtaining data representing use of some or all of the plurality of medical instruments in a plurality of first procedures on a plurality of first patients;
   - storing in a database, for each of the used medical instruments, first data identifying the patient on which the medical instrument has been used;
   - reprocessing the used medical instruments;
   - storing in the database, for each of the reprocessed medical instruments, second data identifying at least one reprocessing modality selected from:

     - a transport device used to transport the medical device before, during, or after reprocessing,
     - a pre-cleaning station in which the medical device has been precleaned, - an automatic reprocessing machine in which the medical device has been reprocessed,

- a drying cabinet in which the medical instrument has been dried,
- a storage cabinet in which the medical instrument has been stored, and
- a person handling the medical instrument during reprocessing;

- determining, for each of the plurality of first patients, a first indicator representing whether a respective first patient carries a problematic pathogen;
- defining, for each reprocessing modality, a first cross-contamination probability factor representing the probability that a problematic pathogen is transferred from a first medical instrument carrying a problematic pathogen to a second medical instrument not carrying the problematic pathogen while the first and second medical instruments share the same reprocessing modality; and
- combining the first indicators, the first data, the second data, and the first cross-contamination probability factors to determine, for each of the reprocessed medical instruments, a contamination probability that the respective medical instrument carries the problematic pathogen.

2. The method of claim 1, wherein the indicator is a binary indicator.

3. The method of claim 1 or 2, further comprising:

- defining, for each reprocessing modality, a reduction factor defining the reduction of a potential load of problematic pathogens while a medical instrument is treated in the respective reprocessing modality; and using the respective reduction factors when determining the contamination probability.

4. The method of any of claims 1 to 3, further comprising:

- defining a first threshold risk level;
- determining, for each of the used medical instruments, whether the contamination probability exceeds the threshold risk level; and
- excluding each medical instrument, for which the contamination probability exceeds the threshold risk level, from further use.

5. The method of any of claims 1 to 4, further comprising:

- obtaining data representing the reuse of some of the medical instruments in a plurality of second procedures on a plurality of second patients;
- storing in the database, for each of the reused medical instruments, third data identifying the patient on which the medical instrument has been reused;
- updating, for each of the plurality of first patients, the first indicator representing whether a respective first patient carries a problematic pathogen;
- combining the updated first indicators, the first data, the second data, and the cross-contamination probability factors to determine, for each of the reused medical instruments, an updated contamination probability at the time of reuse.

6. The method of claim 5, further comprising generating an alert if, for at least one of the reused medical instruments, the updated contamination probability at the time of reuse exceeds the threshold risk level.

7. The method of claim 5 or 6, further comprising:

- defining, for a plurality of procedures offered in a facility, a second cross-contamination probability factor representing the probability that a problematic pathogen is transferred from a first medical instrument carrying a problematic pathogen to a second medical instrument not carrying the problematic pathogen while the first and second medical instruments are used together in the respective procedure;
- reprocessing the reused medical instruments;
- storing in the database, for each of the reprocessed reused medical instruments, fourth data identifying at least one reprocessing modality used on the respective reused instrument; and
- combining the updated first indicators, the first to fourth data, and the first and second cross-contamination probability factors to determine, for each of the reused medical instruments, a further updated contamination probability.

8. An medical instrument provisioning system, comprising:

   - a plurality of medical instruments;
   - a plurality of reprocessing modalities; and
   - a computer comprising a processor, a memory element, and associated hardware;
   wherein machine-readable instructions are stored in the memory element for reading and execution by the processor, and
   wherein the machine-readable instructions cause the processor to execute the method of any one of claims 1 to 7.

Fig. 1

EP 4 654 212 A1

Fig. 2

```
┌─────────────────┐                              ┌─────────────────┐
│ Provide Patient │                              │     Provide     │
│ Data Structure  │                              │    Operation    │
│                 │                              │ Data Structure  │
└─────────────────┘                              └─────────────────┘
         │                                                │
         │                                                │
┌─────────────────┐                              ┌─────────────────┐
│     Provide     │                              │     Provide     │
│   Instrument    │                              │    Operation    │
│ Data Structure  │                              │ Data Structure  │
└─────────────────┘                              └─────────────────┘
         │                                                │
         │                                                │
┌─────────────────┐                              ┌─────────────────┐
│    Determine    │                              │    Determine    │
│  Contamination  │                              │     Cross-      │
│     Matrix      │                              │  Contamination  │
│                 │                              │     Matrix      │
└─────────────────┘                              └─────────────────┘
         │                                                │
         │                                       ┌─────────────────┐
         │                                       │     Update      │
         │                                       │  Contamination  │
         │                                       │     Matrix      │
         │                                       └─────────────────┘
```

## Fig. 3

```
                  ┌─────────────────┐
                  │    For each     │
                  │   Instrument:   │
                  └─────────────────┘
                           │
                           │
                         ╱   ╲
                       ╱       ╲
            No       ╱  Risk >    ╲      Yes
         ◄─────────╱   Threshold    ╲─────────────┐
                   ╲  Risk Level    ╱              ┆
                     ╲     ?      ╱                ┆
                       ╲       ╱                   ┆
                         ╲   ╱                     ┆
                           │                       ┆
   ┌─────────┐        ┌─────────┐          ┌─────────┐
   │  Reuse  │        │ Discard │          │  Issue  │
   │         │        │         │          │  Alert  │
   └─────────┘        └─────────┘          └─────────┘
```

## Fig. 4

Europäisches
Patentamt

European
Patent Office

Office européen
des brevets

# EUROPEAN SEARCH REPORT

**Application Number**

EP 25 17 4880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/189449 A1 (KARUMBA SAMUEL M [KE] ET AL) 5 July 2018 (2018-07-05) * the whole document * | 1-8 | INV. G16H40/40 |
| A | US 2016/235280 A1 (SLY WARD [US] ET AL) 18 August 2016 (2016-08-18) * paragraphs [0002], [0015]; figures 1, 3, 6, 8 * | 1-8 | |
| A | WO 2021/245462 A1 (NANOSONICS LTD [AU]) 9 December 2021 (2021-12-09) * the whole document * | 1-8 | |
| A | RUTALA WILLIAM A ET AL: "Risk of disease transmission to patients from "contaminated" surgical instruments and immediate use steam sterilization", AJIC: AMERICAN JOURNAL OF INFECTION CONTROL, ELSEVIER, AMSTERDAM, NL, vol. 51, no. 11, 25 October 2023 (2023-10-25), XP087430767, ISSN: 0196-6553, DOI: 10.1016/J.AJIC.2023.01.019 [retrieved on 2023-10-25] * abstract; figure 1; table 1 * | 1-8 | TECHNICAL FIELDS SEARCHED (IPC) G16H A61B A61L |
| A | US 2021/386508 A1 (JACKSON MARK [GB] ET AL) 16 December 2021 (2021-12-16) * paragraphs [0002], [0052]; figures 1, 3, 7 * | 1-8 | |
| A | US 2020/179549 A1 (THOMPSON BRIAN J [US] ET AL) 11 June 2020 (2020-06-11) * paragraphs [0074], [0082]; figures 1, 2 * | 1-8 | |

The present search report has been drawn up for all claims

| Place of search | Date of completion of the search | Examiner |
|---|---|---|
| Munich | 24 September 2025 | Lorenz, Larissa |

EPO FORM 1503 03.82 (P04C01)

**EP 4 654 212 A1**

## ANNEX TO THE EUROPEAN SEARCH REPORT
## ON EUROPEAN PATENT APPLICATION NO.

EP 25 17 4880

This annex lists the patent family members relating to the patent documents cited in the above-mentioned European search report.
The members are as contained in the European Patent Office EDP file on
The European Patent Office is in no way liable for these particulars which are merely given for the purpose of information.

24-09-2025

| Patent document cited in search report | | Publication date | Patent family member(s) | | Publication date |
|---|---|---|---|---|---|
| US 2018189449 | A1 | 05-07-2018 | NONE | | |
| US 2016235280 | A1 | 18-08-2016 | CN | 104081411 A | 01-10-2014 |
| | | | EP | 2805287 A1 | 26-11-2014 |
| | | | US | 2015109193 A1 | 23-04-2015 |
| | | | US | 2016235280 A1 | 18-08-2016 |
| | | | US | 2017273547 A1 | 28-09-2017 |
| | | | US | 2019008366 A1 | 10-01-2019 |
| | | | US | 2020029795 A1 | 30-01-2020 |
| | | | WO | 2013109525 A1 | 25-07-2013 |
| WO 2021245462 | A1 | 09-12-2021 | AU | 2021285472 A1 | 09-02-2023 |
| | | | CA | 3181138 A1 | 09-12-2021 |
| | | | EP | 4162506 A1 | 12-04-2023 |
| | | | JP | 2023528495 A | 04-07-2023 |
| | | | US | 2023343448 A1 | 26-10-2023 |
| | | | WO | 2021245462 A1 | 09-12-2021 |
| US 2021386508 | A1 | 16-12-2021 | EP | 3852603 A1 | 28-07-2021 |
| | | | US | 2021386508 A1 | 16-12-2021 |
| | | | US | 2023035411 A1 | 02-02-2023 |
| | | | US | 2024024068 A1 | 25-01-2024 |
| | | | WO | 2020096888 A1 | 14-05-2020 |
| US 2020179549 | A1 | 11-06-2020 | US | 2017252474 A1 | 07-09-2017 |
| | | | US | 2020179549 A1 | 11-06-2020 |
| | | | US | 2023381361 A1 | 30-11-2023 |

EPO FORM P0459

For more details about this annex : see Official Journal of the European Patent Office, No. 12/82